(19) Europäisches Patentamt
European Patent Office
Office européen des brevets

(11) Publication number: 0 308 170 A1

# (12) EUROPEAN PATENT APPLICATION

(21) Application number: 88308439.4

(22) Date of filing: 13.09.88

(51) Int. Cl.4: A 01 N 43/12
A 01 N 43/90, C 07 D 409/12,
C 07 D 493/08, C 07 D 493/10
//(C07D493/08,311:00,311:00),
(C07D493/08,307:00,307:00)

(30) Priority: 15.09.87 US 96442 13.04.88 US 181026

(43) Date of publication of application:
22.03.89 Bulletin 89/12

(84) Designated Contracting States: ES GR

(71) Applicant: E.I. DU PONT DE NEMOURS AND COMPANY
Legal Department 1007 Market Street
Wilmington Delaware 19898 (US)

(72) Inventor: Bozarth, Gene Allen
15 Nathalie Drive
Hockessin Delaware 19707 (US)

Christensen, Joel Robert
46 Choate Street
Newark Delaware 19711 (US)

Powell, James Edward
168 Chandlee Road
Rising Sun Maryland 21911 (US)

(74) Representative: Hildyard, Edward Martin et al
Frank B. Dehn & Co. European Patent Attorneys Imperial
House 15-19 Kingsway
London WC2B 6UZ (GB)

(54) **Selective oxabicycloalkanes.**

(57) A group of oxabicycloalkane herbicides of the formula

$JOCH_2Q$

wherein J is a substituted oxabicycloalkane moiety; and Q is optionally substituted phenyl or substituted thienyl; exhibit selective herbicidal activity, especially in paddy rice. The compounds may be made e.g. by coupling an appropriately substituted thenyl bromide with the appropriate substituted oxabicycloalkanol.

EP 0 308 170 A1

**Description**

## SELECTIVE OXABICYCLOALKANES

### Related Applications

This application is a continuation-in-part of copending application U.S. Serial No. 07/096442 filed September 15, 1987.

### Background of the Invention

This invention relates to compounds, agriculturally suitable compositions thereof, and a method for controlling the growth of undesired vegetaion in paddy rice with an oxabicycloalkane herbicide.

U.S. 4,670,041 and U.S. 4,486,219 disclose oxabicycloalkanes and their use for controlling plant growth.

### Summary of the Invention

This invention comprises a method for controlling the growth of undesired vegetation in a paddy rice crop by applying to the locus of the paddy rice crop an effective amount of a compound of Formula I

$$JOCH_2Q$$

Formula I

wherein

J is 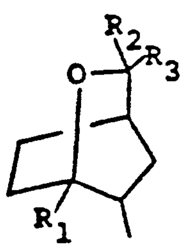 , 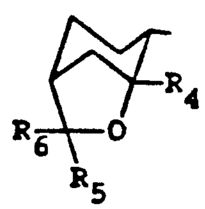 or 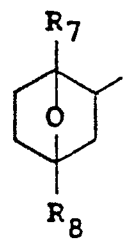 ;

J-1 J-2 J-3

$R_1$ is $CH_3$ or $CH_2CH_3$;
$R_2$ and $R_3$ are independently $CH_3$ or $CH_2CH_3$ or
$R_2$ and $R_3$ may be taken together as $-(CH_2)_n-$where n is 4 or 5;
$R_4$ is $CH_3$ or $CH_2CH_3$;
$R_5$ and $R_6$ are independently $CH_3$ or $CH_2CH_3$;
$R_7$ is $CH_3$ or $CH_2CH_3$;
$R_8$ is $CH_3$, or $CH_2CH_3$ or $CH(CH_3)_2$;

Q is 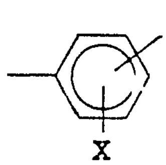 , 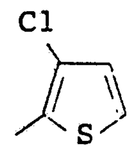 ,

Q-1 Q-2

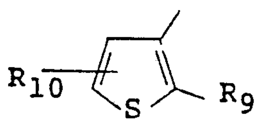 or 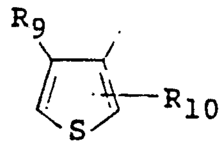 ;

Q-3 Q-4

X and Y are independently H, F, Cl or $CH_3$;
$R_9$ is F, Cl, Br, $CH_3$ or $CH_2CH_3$; and
$R_{10}$ is H, F, Cl or $CH_3$;
provided that when J is J-3, then Q is Q-3 or Q-4.

Preferred for reasons of more efficient weed control and/or better crop tolerance are:

1. The method wherein
$R_1$ is $CH_3$;
$R_2$ and $R_3$ are $CH_2CH_3$ or $R_2$ and $R_3$ are taken together as $-(CH_2)_n-$ where n is 4 or 5;
$R_4$ is $CH_3$;
$R_5$ and $R_6$ are $CH_3$;
$R_7$ is $CH_3$; and
$R_8$ is $CH(CH_3)_2$.
2. The method wherein the crop is transplanted japonica rice.
3. The method wherein the crop is transplanted indica rice.
4. The method wherein among the weeds controlled is barnyardgrass.

Specifically preferred for reasons of most efficient weed control and/or better crop tolerance is the method wherein the compound of Formula I is selected from the group consisting of:
endo-4-[(2-fluorophenyl)methoxy]-5,7,7-trimethyl-6-oxabicyclo[3.2.1]octane;
6-endo-benzyloxy-1-methyl-3,3-tetramethylene-2-oxabicyclo[2.2.2]octane;
6-endo-benzyloxy-1-methyl-3,3-pentamethylene-2-oxabicyclo[2.2.2]octane;
6-endo-[(2-fluorophenyl)methoxy]-3,3-pentamethylene-2-oxabicyclo[2.2.2]octane;
1-methyl-3,3-diethyl-6-endo-[(2-fluorophenyl)methoxy]-2-oxabicyclo[2.2.2]octane;
exo-2-[(2-bromo-3-thienyl)methoxy]-1-methyl-4-(1-methylethyl)-7-oxabicyclo[2.2.1]heptane;
exo-2-[(2-ethyl-3-thienyl)methoxy]-1-methyl-4-(1-methylethyl)-7-oxabicyclo[2.2.1]heptane; and
exo-2-[(2-methyl-3-thienyl)methoxy]-1-methyl-4-(1-methylethyl)-7-oxabicyclo[2.2.1]heptane.

This invention also comprises compounds of Formula I wherein Q is Q-3 or Q-4 and agriculturally suitable compositions containing them.

Preferred compounds of the invention for reasons of more efficient weed control and/or better crop tolerance are:

1. The compounds of Formula I wherein
$R_1$ is $CH_3$;
$R_2$ and $R_3$ are $CH_2CH_3$ or $R_2$ and $R_3$ are taken together as $-(CH_2)_n$ where n is 4 or 5;
$R_4$ is $CH_3$;
$R_5$ and $R_6$ are $CH_3$;
$R_7$ is $CH_3$; and
$R_8$ is $CH(CH_3)_2$.
2. The compounds of Preferred 1 wherein J is J-3.

Specifically preferred compounds of the invention for reasons of most efficient weed control and/or better crop tolerance are compounds of Formula I selected from the group consisting of:
exo-2-[(2-bromo-3-thienyl)methoxy]-1-methyl-4-(1-methylethyl)-7-oxabicyclo[2.2.1]heptane;
exo-2-[(2-ethyl-3-thienyl)methoxy]-1-methyl-4-(1-methylethyl)-7-oxabicyclo[2.2.1]heptane; and

exo-2-[(2-methyl-3-thienyl)methoxy]-1-methyl-4-(1-methylethyl)-7-oxabicyclo[2.2.1]heptane.

## DETAILED DESCRIPTION OF THE INVENTION

Many of the compounds of Formula I are known in the art. They can be prepared according to processes described in U.S. 4,670,041 and U.S. 4,486,219.

Compounds of Formula I wherein Q is Q-3 and $R_{10}$ is H, F or Cl can be prepared as described in Scheme 1.

SCHEME I

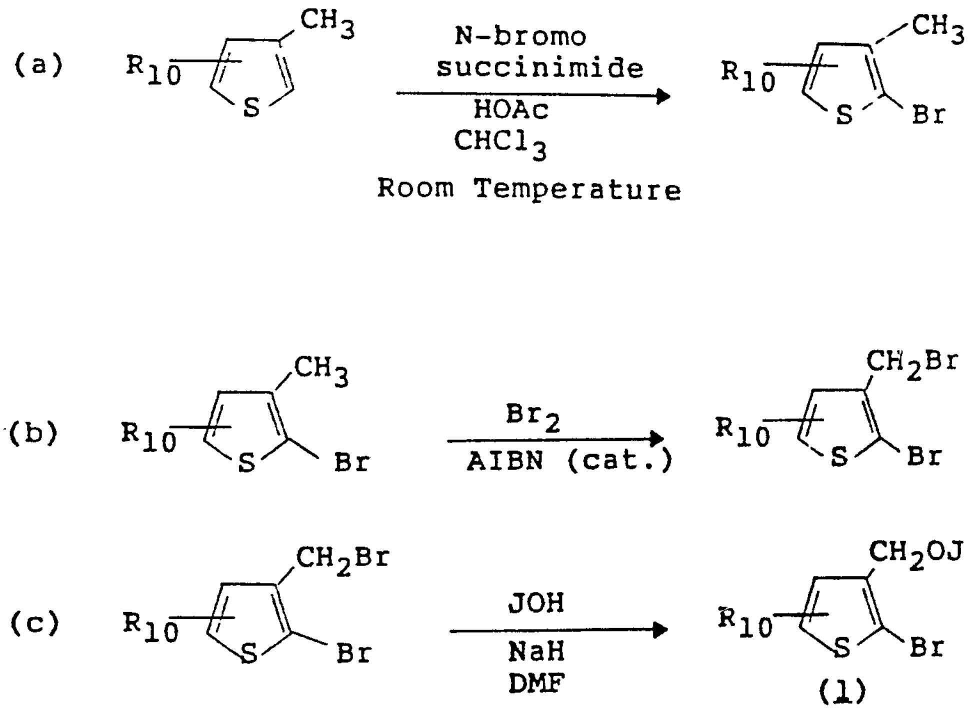


The bromide (1) can then be converted to compounds of Formula I wherein $R_9$ is $CH_3$, $CH_2CH_3$, F or Cl by lithiation, for example, with n-butyl lithium at -78° C followed by treatment with a methylating or ethylating agent or an anion fluorinating or chlorinating agent which are well known to one skilled in the art. When $R_{10}$ is $CH_3$, following the bromination with $Br_2$(AIBN), Scheme 1b, the desired product is first separated from the dibromo derivatives or the alternate bromo methyl isomers by methods well known to one skilled in the art (e.g. chromatography), prior to reaction with JOH (Scheme 1c).

Compounds of Formula I where Q is Q-4 and R10 is H, F or Cl can be prepared as described in Scheme 2.

SCHEME 2

(a) 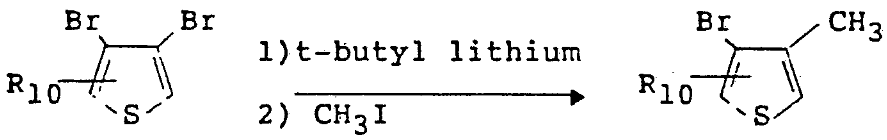


(b) 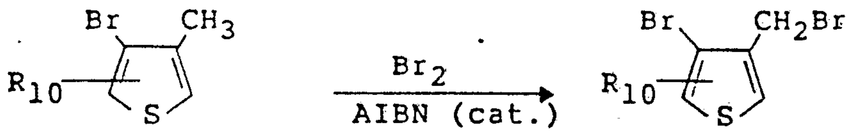


(c) 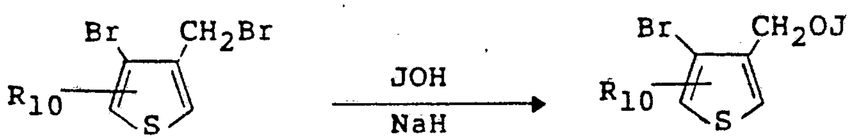


(2)

Again, the bromide (2) can then be converted to compounds of Formula I wherein $R_9$ is $CH_3$, $CH_2CH_3$, F or Cl by lithiation followed by treatment with an alkylating or halogenating agent. When $R_{10}$ is $CH_3$, the separation of the desired product of the bromination of Scheme 2b from dibromination and isomeric products will also be required prior to carrying out the reaction of Scheme 2c.

All stereoisomers (diastereomers and enantiomers); endo and exo forms; and mixtures thereof are included within the scope of the present invention.

The various individual isomeric forms and various combinations of the derivatives usually have some difference in herbicidal or plant growth control properties.

The following examples serve to illustrate the preparation of compounds of the invention:

Example 1

2-Bromo-3-Methylthiophene

To a solution containing 600 ml of chloroform, 600 ml of acetic acid, and 98.0 g of 3-methylthiophene was added portionwise 188.0g of N-Bromosuccinimide with stirring. The addition was exothermic and the reaction mixture was kept below 35°C by means of an ice bath. After the addition was complete, the reaction was allowed to sit for 30 minutes at room temperature whereupon 1.2L of water was added. The organic layer was separated, washed with 600ml of water, followed by 600 ml of 1N NaOH, and finally 600 ml of water. The organic layer was dried over $MgSO_4$, filtered, and the filtrate reduced to a tan oil. Fractional distillation afforded 128g of a yellow mobile oil boiling between 130°C and 170°C.

Example 2

2-Bromo-3-Bromomethylthiophene

To a solution of 120.0g of 2-bromo-3-methylthiophene in 1000ml of carbon tetrachloride was added 0.5g of Azobisisobutyronitrile. The solution was heated to reflux and 34.9 ml of bromine ($Br_2$(1)) was added dropwise over a three hour period. After continued refluxing for 30 minutes, the mixture was cooled to room

temperature, and washed with 500 ml portions of water, saturated $NaHSO_3$(aq) and saturated $NaHCO_3$ (aq). The organic layer was dried over $MgSO_4$, filtered, and the filtrate reduced to a dark oil. Vacuum distillation afforded 86.8g of a yellow oil boiling between 80 and 92° C at 2 mm. The material was estimated to be 90% pure.

Example 3

exo-2-[(2-bromo-3-thienyl)methoxy]-1-methyl-4-(1-methylethyl)-7-oxabicyclo[2.2.1]heptane

Sodium hydride (60% in oil, 13,5g) was washed with 30 ml of dry hexane under a nitrogen atmosphere. To the washed sodium hydride was added 500 ml of anhydrous dimethylformamide followed by 52.0g of (±)-2-exo-hydroxy-1-methyl-4-isopropyl-7-oxabicyclo[2.2.1]heptane. The resulting mixture was stirred for 2 hours at the end of which $H_2$(g) evolution had ceased. The mixture was cooled to -10° C and 86.8g of 2-bromo-3-bromomethyl thiophene was added portionwise keeping the temperature below 0° C. After the addition was complete, the mixture was allowed to warm to room temperature and stirred overnight. The mixture was poured into 500ml of ice-water, extracted 3 times with ether and the combined ether extracts were washed 3 times with 200ml portions of water, dried over $MgSO_4$, filtered and the filtrate concentrated to afford 110g of a brown oil. Flash chromatography over silica gel using 15% ethylacetate in hexane yielded 93g of a light brown oil.
NMR($^1$H, 200MHz, $CDCl_3$, ppm)
(d - 7.2, 1H, J=6Hz, ArH)
(d - 7.0, 1H, J=6Hz, Arh)
(AB quartet, 4.4, 2H, J=11Hz, OCH2)
(m, 3.5, 1H, CH-0-)
(s, 1.45, 3H, $CH_3$)
(dd, 0.97, 6H, $CH(CH_3)_2$)
(m, 1.9-2.2, 3H Aliphatic ring H's)
(m, 1.4-1.6, 4H Aliphatic ring H's)

Example 4

exo-2-[(2-methyl-3-thienyl)methoxy]-1-methyl-4-(1-methylethyl)-7-oxabicyclo[2.2.1]heptane

To 1.5g (4.5 mmoles) of the compound of Example 3 in 25 ml of dry tetrahydrofuran, cooled to -78° C, was added dropwise 2.0 ml of 2.55M n-butyl lithium in hexane, while maintaining the reaction temperature below -55° C. Stirring was continued for an additional 10 minutes at -78° C, then 0.32 ml of methyl iodide was added and the mixture allowed to warm to room temperature and stirred overnight. The reaction mixture was poured into 100 ml of ice-water, extracted with 150 ml of ether, washed with water, dried over $MgSO_4$, filtered and concentrated to yield 0.90g of a light yellow oil. It was shown to be 90% pure by NMR.
NMR($^1$H, 200MHz, $CDCl_3$, ppm)
(d - 7.0, 1H, J=$CH_3$, ArH)
(d - 6.93, 1H, J=6Hz, ArH
(q - 4.4, 2H, $OCH_2$)
(m - 3.5, 1H, CH-0-)
(s - 2.42, 3H, -$CH_3$)
(s - 1.44, 3H, -$CH_3$)
(dd - 0.97, 6H, $CH(CH_3)_2$)
(m - 1.4-2.3, 7H, Aliphatic ring H's)

Formulations

The method of this invention can be conveniently carried out by formulating a compound of Formula I in the conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The herbicidal formulations of the invention comprise 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, micro-biological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and J. D. Fryer and S. A. Evans, "Weed Control Handbook", 5th Ed., Blackwell Scientific Publications, Oxford, 1968, pp. 101-103.

In the following Examples, all parts are by weight unless otherwise indicated.

Example 5

Wettable Powder
endo-4-[(2-fluorophenyl)methoxy]-5,7,7-trimethyl-6-oxabicyclo[3.2.1]octane 50%
sodium alkylnaphthalenesulfonate 2%
low viscosity methyl cellulose 2%
diatomaceous earth 46%

The ingredients are blended, coarsely hammer-milled and then air-milled to produce particles essentially all below 10 microns in diameter. The product is reblended before packaging.

Example 6

Granule
Wettable Powder of Example 5 5%
attapulgite granules (U.S.S. 20 to 40 mesh; 0.84-0.42 mm) 95%

A slurry of wettable powder containing 25% solids is sprayed on the surface of attapulgite granules in a double-cone blender. The granules are dried and packaged.

Example 7

Extruded Pellet
endo-4-[(2-fluorophenyl)methoxy]-5,7,7-trimethyl-6-oxabicyclo[3.2.1]octane 25%
anhydrous sodium sulfate 10%
crude calcium ligninsulfonate 5%
sodium alkylnaphthalenesulfonate 1%
calcium/magnesium bentonite 59%

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 8

Low Strength Granule
endo-4-[(2-fluorophenyl)methoxy]-5,7,7-trimethyl-6-oxabicyclo[3.2.1]octane 0.1%
attapulgite granules (U.S.S. 20 to 40 mesh) 99.9%

The solvent ingredient is dissolved in a solvent and the solution is sprayed upon dedusted granules in a double-cone blender. After spraying of the solution has been completed, the material is warmed to evaporate the solvent. The material is allowed to cool and then packaged.

Example 9

Low Strength Granule
endo-4-[(2-fluorophenyl)methoxy]-5,7,7-trimethyl-6-oxabicyclo[3.2.1]octane 1%
N,N-dimethylformamide 9%
attapulgite granules (U.S.S. 20 to 40 sieve) 90%

The active ingredient is dissolved in the solvent and the solution is sprayed upon dedusted granules in a double cone blender. After spraying of the solution has been completed, the blender is allowed to run for a short period and then the granules are packaged.

Example 10

Wettable Powder

endo-4-[(2-fluorophenyl)methoxy]-5,7,7-trimethyl-6-oxabicyclo[3.2.1]octane 40%
sodium ligninsulfonate 20%
montmorillonite clay 40%

The ingredients are thoroughly blended, coarsely hammer-milled and then air-milled to produce particles essentially all below 10 microns in size. The material is reblended and then packaged.

Example 11

Emulsifiable Concentrate

endo-4-[(2-fluorophenyl)methoxy]-5,7,7-trimethyl-6-oxabicyclo[3.2.1]octane 35%
blend of polyalcohol carboxylic esters and oil soluble petroleum sulfonates 6%
xylene 59%

The ingredients are combined and filtered to remove undissolved solids. The product can be used directly, extended with oils, or emulsified in water.

Example 12

Dust

endo-4-[(2-fluorophenyl)methoxy]-5,7,7-trimethyl-6-oxabicyclo[3.2.1]octane 10%
attapulgite 10%
Pyrophyllite 80%

The active ingredient is blended with attapulgite and then passed through a hammer-mill to produce particles substantially all below 200 microns. The ground concentrate is then blended with powdered pyrophyllite until homogeneous.

Example 13

Wettable Powder

endo-4-[(2-fluorophenyl)methoxy]-5,7,7-trimethyl-6-oxabicyclo[3.2.1]octane 20%
sodium alkylnaphthalenesulfonate 4%
sodium ligninsulfonate 4%
low viscosity methyl cellulose 3%
attapulgite 69%

The ingredients are thoroughly blended. After grinding in a hammer-mill to produce particles essentially all below 100 microns, the material is reblended and sifted through a U.S.S. No. 50 sieve (0.3 mm opening) and packaged.

Utility

Test results indicate that the compounds of the present invention are highly active preemergent or postemergent herbicides or plant growth regulants. They have utility for selective weed control in paddy rice fields containing japonica or indica rice.

Compounds of this invention are particularly useful for the control of weeds in paddy rice. They may be applied postemergence to paddy rice or to rice from which the flood has been removed. They may also be applied to paddy rice after transplanting as a spray or granule. The application may be made from 3 to 10 days after transplanting.

Rates of 16 to 1500 g/ha will provide weed control. The compounds are particularly useful for the control of barnyardgrass (Echinochloa crus-galli), a pernicious weed in rice culture, but may also provide complete or partial control of other weeds, particularly gramineous weeds, in rice.

The compounds of this invention may be used in combination with other commercial herbicides. They are particularly useful in combination with the following herbicides:

methyl 2-[[[[(4,6-dimethoxy-2-pyrimidinyl) amino]carbonyl]amino]sulfonyl]methyl benzoate (bensulfuron methyl)

ethyl 5-[3-(4,6-dimethoxypyrimidin-2-yl)ureidosulfonyl]-1-methylpyrazole-4-carboxylate

Compounds

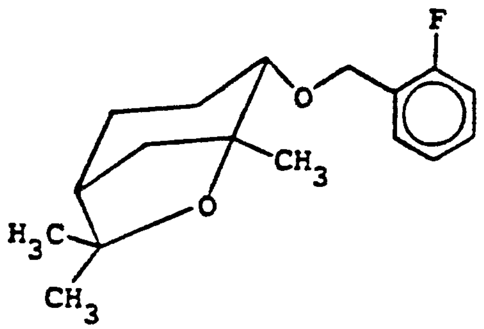

Compound 1

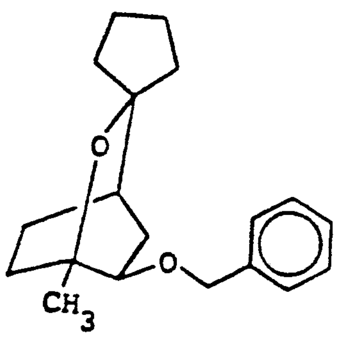

Compound 2

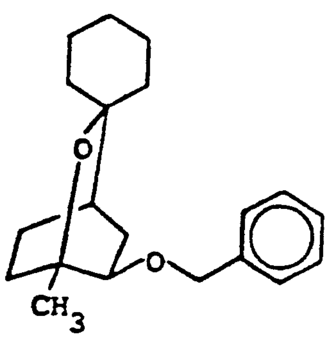

Compound 3

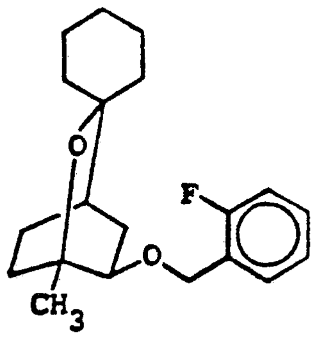

Compound 4

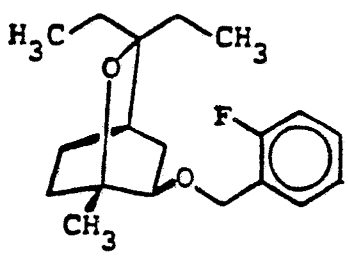

Compound 5

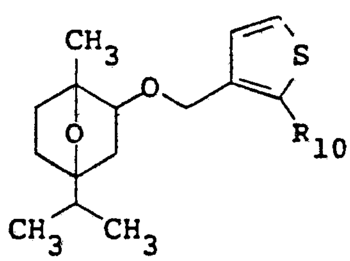

| Compound | $R_{10}$ |
|---|---|
| 6 | Br |
| 7 | $CH_3$ |
| 8 | $CH_2CH_3$ |

Example 14

Waxed paper cups with a top surface area of 100 $cm^2$ were partially filled with Tama silt loam soil. Either barnyardgrass or japonica rice seeds were planted into the soil in each pot, which was kept moist until the seedlings had reached the one leaf stage. Water was then added to a 3 cm depth and applications of chemical were made directly to the water after being formulated in a non-phytoxic solvent. Treatments were made at the 1.5-2.0 leaf stage of barnhardgrass and 1 leaf stage of rice. Visual evaluations of weed control and crop injury were made 14 days after application. The test results are summarized in Table 1.

Table 1

| COMPOUND | RATE g/ha | % INJURY OR CONTROL | |
|---|---|---|---|
| | | RICE | BARNYARD GRASS |
| 1 | 8 | - | 10 |
| | 32 | 19 | 75 |
| | 125 | 0 | 95 |
| | 500 | 31 | 98 |
| | 1000 | 68 | - |
| 2 | 8 | - | 10 |
| | 32 | 4 | 50 |
| | 125 | 13 | 90 |
| | 500 | 5 | 98 |
| | 1000 | 24 | - |
| 3 | 8 | - | 0 |
| | 32 | 0 | 90 |
| | 125 | 0 | 98 |
| | 500 | 1 | 100 |
| | 1000 | 3 | - |
| 4 | 8 | - | 0 |
| | 32 | 0 | 90 |
| | 125 | 5 | 100 |
| | 500 | 0 | 100 |
| | 1000 | 0 | - |
| 5 | 8 | - | 0 |
| | 32 | 8 | 30 |
| | 125 | 0 | 90 |
| | 500 | 23 | 100 |
| | 1000 | 0 | - |
| 6 | 16 | - | 27 |
| | 32 | 0 | 60 |
| | 64 | 0 | 92 |
| | 125 | 10 | 98 |
| | 250 | 40 | 98 |
| | 500 | 67 | - |
| 7 | 16 | - | 40 |
| | 32 | 33 | 67 |
| | 64 | 63 | 90 |
| | 125 | 80 | 95 |
| | 250 | 97 | 98 |
| | 500 | 98 | - |

Table 1 (continued)

| | | | |
|---|---|---|---|
| 8 | 16 | - | 17 |
| | 32 | 0 | 47 |
| | 64 | 47 | 78 |
| | 125 | 50 | 86 |
| | 250 | 70 | 97 |
| | 500 | 93 | - |

## Claims

1. The method for controlling the growth of undesired vegetation in a paddy rice crop which comprises applying to the locus of the paddy rice crop an effective amount of a compound of the formula:

$$JOCH_2Q$$

I

wherein

J is 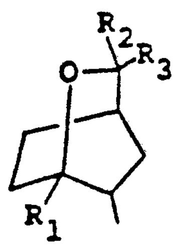 , 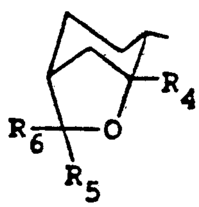 or 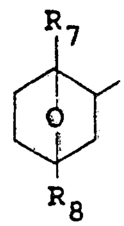 ;

J-1 J-2 J-3

$R_1$ is $CH_3$ or $CH_2CH_3$;
$R_2$ and $R_3$ are independently $CH_3$ or $CH_2CH_3$ or
$R_2$ and $R_3$ may be taken together as $-(CH_2)_n-$where n is 4 or 5;
$R_4$ is $CH_3$ or $CH_2CH_3$;
$R_5$ and $R_6$ are independently $CH_3$ or $CH_2CH_3$;
$R_7$ is $CH_3$ or $CH_2CH_3$;
$R_8$ is $CH_3$, or $CH_2CH_3$ or $CH(CH_3)_2$;

Q is 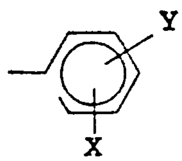 , 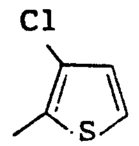 ,

Q-1 Q-2

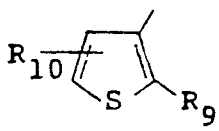 or 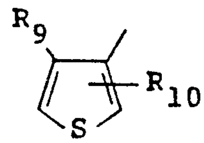 ;

Q-3 Q-4

X and Y are independently H, F, Cl or $CH_3$;
$R_9$ is F, Cl, Br, $CH_3$ or $CH_2CH_3$; and
$R_{10}$ is H, F, Cl or $CH_3$;
provided that when J is J-3, then Q is Q-3 or Q-4.

2. The method of Claim 1 wherein
$R_1$ is $CH_3$;
$R_2$ and $R_3$ are $CH_2CH_3$ or $R_2$ and $R_3$ are taken together as $-(CH_2)_n-$ where n is 4 or 5;
$R_4$ is $CH_3$;
$R_5$ and $R_6$ $CH_3$;
$R_7$ is $CH_3$; and
$R_8$ is $CH(CH_3)_2$.

3. The method of Claim 1 wherein the crop is transplanted japonica rice.

4. The method of Claim 1 wherein the crop is transplanted indica rice.

5. The method of Claim 1 wherein among the weeds controlled is barnyardgrass.

6. The method of Claim 1 wherein the compound is endo-4-[(2-fluorophenyl)methoxy]-5,7,7-trimethyl-6-oxabicylco[3.2.1]octane.

7. The method of Claim 1 wherein the compound is 6-endo-benzyloxy-1-methyl-3,3-tetramethylene-2-oxabicyclo[2.2.2]octane

8. The method of Claim 1 wherein the compound is 6-endo-benzyloxy-1-methyl-3,3-pentamethylene-2-oxabicyclo[2.2.2]octane

9. The method of Claim 1 wherein the compound is 6-endo-[(2-fluorophenyl)methoxy]-3,3-pentamethylene-2-oxabicyclo[2.2.2]octane

10. The method of Claim 1 wherein the compound is 1-methyl-3,3-diethyl-6-endo-[(2-fluorophenyl)methoxy]-2-oxabicyclo[2.2.2]octane

11. The method of Claim 1 wherein the compound is exo-2-[(2-bromo-3-thienyl)methoxy]-1-methyl-4-(1-methylethyl)-7-oxabicyclo[2.2.1]heptane

12. The method of Claim 1 wherein the compound is exo-2-[(2-ethyl-3-thienyl)methoxy]-1-methyl-4-(1-methylethyl)-7-oxabicyclo[2.2.1]heptane

13. The method of Claim 1 wherein the compound is exo-2-[(2-ethyl-3-thienyl)methoxy]-1-methyl-4-(1-methylethyl)-7-oxabicyclo[2.2.1]heptane.

14. A compound of the formula:

$JOCH_2Q$

I

wherein

J is 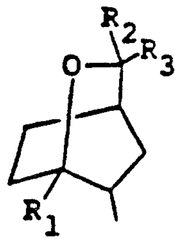 , 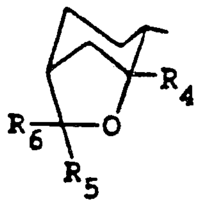 or 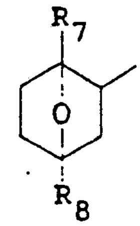 ;

J-1 J-2 J-3

$R_1$ is $CH_3$ or $CH_2CH_3$;
$R_2$ and $R_3$ are independently $CH_3$ or $CH_2CH_3$ or
$R_2$ and $R_3$ may be taken together as $-(CH_2)_n-$where n is 4 or 5;
$R_4$ is $CH_3$ or $CH_2CH_3$;
$R_5$ and $R_6$ are independently $CH_3$ or $CH_2CH_3$;
$R_7$ is $CH_3$ or $CH_2CH_3$;
$R_8$ is $CH_3$, or $CH_2CH_3$ or $CH(CH_3)_2$;

Q is 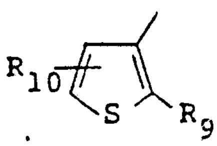 or 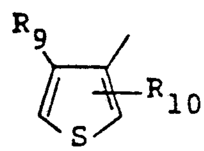 ;

Q-3 Q-4

$R_9$ is F, Cl, Br, $CH_3$ or $CH_2CH_3$; and
$R_{10}$ is H, F, Cl or $CH_3$.

15. A compound of Claim 14 wherein
$R_1$ is $CH_3$;
$R_2$ and $R_3$ are $CH_2CH_3$ or $R_2$ and $R_3$ are taken together as $-(CH_2)_n-$ where n is 4 or 5;
$R_4$ is $CH_3$;
$R_5$ and $R_6$ are $CH_3$;
$R_7$ is $CH_3$; and
$R_8$ is $CH(CH_3)_2$.

16. A compound of Claim 15 wherein J is J-3.

17. The compound of Claim 16 which is exo-2-[(2-bromo-3-thienyl)methoxy]-1-methyl-4-(1-methylethyl)-7-oxabicyclo[2.2.1]heptane.

18. The compound of Claim 16 which is exo-2-[(2-ethyl-3-thienyl)methoxy]-1-methyl-4-(1-methylethyl)-7-oxabicyclo[2.2.1]heptane.

19. The compound of Claim 16 which is exo-2-[(2-methyl-3-thienyl)methoxy]-1-methyl-4-(1-methylethyl)-7-oxabicyclo[2.2.1]heptane.

20. A composition suitable for controlling the growth of undesired vegetation which comprises an effective amount of a compound of Claim 14 and at least one of the following: surfactant, solid or liquid diluent.

21. A composition suitable for controlling the growth of undesired vegetation which comprises an effective amount of a compound of Claim 15 and at least one of the following: surfactant, solid or liquid diluent.

22. A composition suitable for controlling the growth of undesired vegetation which comprises an effective amount of a compound of Claim 16 and at least one of the following: surfactant, solid or liquid diluent.

23. A composition suitable for controlling the growth of undesired vegetation which comprises an effective amount of a compound of Claim 17 and at least one of the following: surfactant, solid or liquid

diluent.

24. A composition suitable for controlling the growth of undesired vegetation which comprises an effective amount of the compound of Claim 18 and at least one of the following: surfactant, solid or liquid diluent.

25. A composition suitable for controlling the growth of undesired vegetation which comprises an effective amount of the compound of Claim 19 and at least one of the following: surfactant, solid or liquid diluent.

26. A composition suitable for controlling the growth of undesired vegetation in the locus of a rice paddy which comprises an effective amount of a compound of Claim 14 and at least one of the following: surfactant, solid or liquid diluent.

European Patent Office

# EUROPEAN SEARCH REPORT

Application Number

EP 88 30 8439

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 081 893 (SHELL INTERNATIONALE RESEARCH MAATSCHAPPIJ) * Claims; page 4, lines 20-22 * & US-A-4 670 041 (Cat. D) | 1-26 | A 01 N 43/12<br>A 01 N 43/90<br>C 07 D 409/12<br>C 07 D 493/08<br>C 07 D 493/10 //<br>(C 07 D 493/08<br>C 07 D 311:00<br>C 07 D 311:00 )<br>(C 07 D 493/08<br>C 07 D 307:00<br>C 07 D 307:00 ) |
| X,D | US-A-4 486 219 (J.E.POWELL) * Claims; column 1, lines 57-68 * | 1-6,14-16,20-22,26 | |
| X | GB-A-2 188 931 (SHELL INTERNATIONALE RESEARCH MAATSCHAPPIJ) * Claims * | 1,14,20,26 | |

TECHNICAL FIELDS SEARCHED (Int. Cl.4)

A 01 N
C 07 D

The present search report has been drawn up for all claims

| Place of search | Date of completion of the search | Examiner |
|---|---|---|
| THE HAGUE | 15-12-1988 | DECORTE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document